# EUROPEAN PATENT SPECIFICATION

(11) **EP 1 808 123 B1**
(45) Date of publication and mention of the grant of the patent: **16.02.2011**
(21) Application number: 05788055.1
(22) Date of filing: 03.10.2005
(51) Int. Cl.: A61B 5/022, A61B 5/0245

(54) **BLOOD PRESSURE MEASURING DEVICE AND BLOOD PRESSURE MEASURING METHOD**
BLUTDRUCKMESSGERÄT UND BLUTDRUCKMESSVERFAHREN
DISPOSITIF DE MESURE DE LA PRESSION ARTERIELLE ET PROCEDE DE MESURE DE LA PRESSION ARTERIELLE

(30) Priority: 06.10.2004 JP 2004294307; 06.10.2004 JP 2004294308
(43) Date of publication of application: 18.07.2007
(73) Proprietor: TERUMO KABUSHIKI KAISHA, Tokyo 151-0072 (JP); NIPPON TELEGRAPH AND TELEPHONE CORPORATION, Tokyo 100-8116 (JP)
(72) Inventor: HABU, Yoshiyuki, Ashigarakami-gun, Kanagawa 2590151 (JP); HAGI, Kouji, Ashigarakami-gun, Kanagawa 2590151 (JP); OZAWA, Hitoshi, Fujinomiya-shi, Shizuoka 4180015 (JP); AIHARA, Kimihisa NIPPON TELEGRAPH & TELEPHONE CORP, Tokyo 1008116 (JP); TATARA, Naoe NIPPON TELEGRAPH & TELEPHONE CORP., Tokyo 1008116 (JP); MINO, Shinji NIPPON TELEGRAPH & TELEPHONE CORP., Tokyo 1008116 (JP); KOIZUMI, Hiroshi NIPPON TELEGRAPH & TELEPHONE CORP, Tokyo 1008116 (JP)
(74) Representative: Paget, Hugh Charles Edward
(86) International application number: PCT/JP2005/018293
(87) International publication number: WO 2006/038589

(56) References cited:
- EP-A- 0 073 123
- EP-A- 1 212 979
- EP-A- 1 424 038
- JP-A- 4 256 727
- JP-A- 4 259 448
- JP-A- 5 146 415
- JP-A- 7 241 279
- JP-A- 11 009 563
- JP-A- 2001 008 909
- JP-A- 2002 172 095
- US-A1- 2003 013 976

## Description

### Technical Field

The present invention relates to a technique which makes it possible to derive blood pressures with high accuracy, particularly in blood pressure measurement of an external ear and its surroundings.

### Background Art

Conventional blood pressure measuring apparatuses which use pulse waves are roughly classified into the photoplethysmography type, pressure plethysmography type, and Korotkoff type, according to their measurement principles. With the photoplethysmography type, light reflected by blood flowing through a part squeezed by a cuff is obtained as a pulse wave signal by a photosensor. With the pressure plethysmography type, the oscillation of blood vessel walls caused by blood flowing through a part squeezed by a cuff is obtained as a pulse wave signal by a pressure sensor. With the Korotkoff type, Korotkoff sounds produced due to squeezing by a cuff are obtained as a pulse wave signal by a microphone installed near the cuff. Blood pressure is measured as the variation of the obtained pulse wave signal with time.

However, under any of the above described measurement principles, measurement noise can occur as a direct or indirect result of body movements. Thus, a method has been proposed which takes measurements by using multiple measurement systems based on different measurement principles or by switching among them, and selects the most probable result based on human judgment, as described in Patent Document 1.

Also, when the pulse wave signal is weak or greatly saturated for some reason, making it impossible to take proper blood pressure measurements, an error signal is generated, prompting the user to change the mounting position of a pulse wave detection sensor before applying pressure and taking blood pressure measurements again or, as described in Patent Document 2, a signal level is adjusted by means of signal amplification or the like before applying pressure and taking blood pressure measurements again.
Patent Document 1: Japanese Patent No. 3240324
Patent Document 2: Japanese Publication of Examined Patent Application No. 6-18555

EP-A-1212979 describes a pulse wave measuring apparatus having a pressure sensor of pulse waves and a photoelectric sensor of pulse waves. The apparatus also has a body motion sensor, whose output is used to remove motion components from the output of the photoelectric sensor.

JP-A-4-259448 describes an electronic blood pressure measuring apparatus for a finger which has a photoelectric pulse wave detector and a pressure pulse wave detector. When an accurate photoelectric pulse wave cannot be detected by reason of the influence of a disturbing light on the photoelectric pulse wave, an out-of-the-way position of a photoelectric sensor and the like, the result of the measurement is judged to be inaccurate, and is not adopted. The process of deciding the blood pressure is then executed in accordance with the information on the pressure pulse wave detected by the pressure pulse wave detector.

### Disclosure of Invention

Conventionally, however, if a proper pulse wave signal is not obtained, it is necessary to take measurements again, for example, after changing the cuff position. This is troublesome. This imposes a physical burden on the patient, who must go through multiple measurement operations, and be squeezed by the cuff multiple times.

The present invention has been made in view of the above problems and has as an object to provide a blood pressure measuring apparatus and blood pressure measuring method which make it possible to obtain a proper pulse wave signal for high-accuracy blood pressure measurements, thereby saving the trouble of taking measurements repeatedly and reducing the physical burden imposed on the user.

The blood pressure measuring apparatus of the invention is set out in claim 1.
The blood pressure measuring method of the invention is set out in claim 2.

### Effect of the Invention

The present invention provides a technique which makes it possible to easily obtain a proper pulse wave signal for blood pressure measurements.

Other features and advantages of the present invention will be apparent from the following description taken in conjunction with the accompanying drawings, in which like reference numerals denote the same or similar parts throughout the figures thereof.

### Brief Description of Drawings

The accompanying drawings, which are incorporated herein and form part of the specification, illustrate embodiments of the present invention and, together with the description, serve to explain the principles of the present invention.
Figure 1 is an internal block diagram of a blood pressure measuring apparatus according to a first embodiment;
Figure 2 is a diagram showing structure and operation in a cuff;
Figure 3 is an external perspective view of the blood pressure measuring apparatus according to the first embodiment;
Figure 4A is an operation flowchart of the blood pressure measuring apparatus according to the first embodiment;
Figure 4B is an operation flowchart of the blood pressure measuring apparatus according to the first embodiment;
Figure 5 is a diagram showing exemplary choices of pulse waves based on a characteristic of body movements for a blood pressure measuring apparatus according to a second embodiment;
Figure 6 is an internal block diagram of a blood pressure measuring apparatus according to a third embodiment;
Figure 7 is a diagram showing a cuff attached to and around a tragus;

Preferred embodiments of the present invention will be described in detail below in an exemplary fashion with reference to the drawings. However, the components described in the embodiments are only exemplary and are not intended to limit the scope of the present invention.

### (First embodiment)

A first embodiment of a blood pressure measuring apparatus according to the present invention will be described by citing a sphygmomanometer which uses a tragus and its surroundings as a measurement site.

### <Equipment configuration>

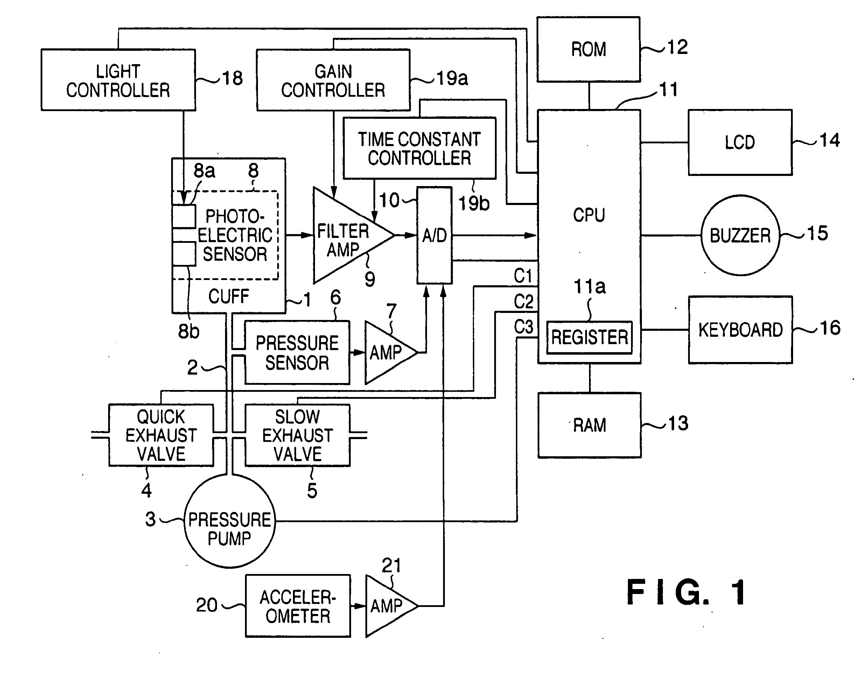

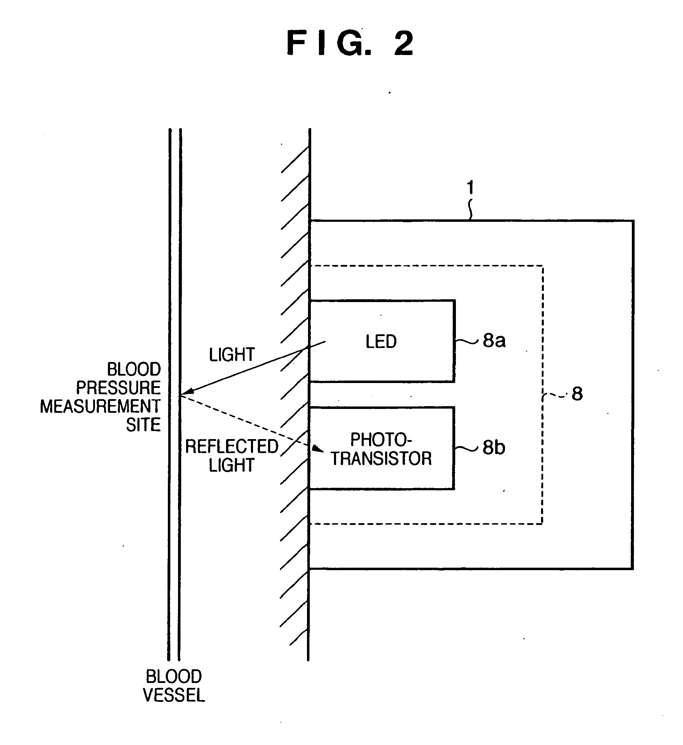

Figure 1 is an internal block diagram of a blood pressure measuring apparatus according to the first embodiment. Figure 2 is a diagram showing structure and operation in a cuff.

Reference numeral 1 denotes a cuff which is secured to a blood pressure measurement site so that it can squeeze blood vessels. Reference numeral 2 denotes a rubber tube which constitutes an air flow path into the cuff 1. Reference numeral 3 denotes a pressure pump which delivers compressed air into the cuff 1. Reference numeral 4 denotes a quick exhaust valve which reduces pressure in the cuff 1 quickly. Reference numeral 5 denotes a slow exhaust valve which reduces pressure in the cuff 1 at a constant rate (2 to 3 mmHg/sec). Reference numeral 6 denotes a pressure sensor which varies an electrical parameter according to the pressure in the cuff 1. Reference numeral 7 denotes a pressure pulse wave detection amplifier (AMP) which detects the electrical parameter from the pressure sensor 6, converts it into an electrical signal, amplifies it, and outputs an analog cuff pressure signal P.

Reference numeral 8 denotes a pulse wave sensor installed in the cuff 1. The pulse wave sensor 8 includes an LED 8a which illuminates pulsating vascular blood flow with light and a phototransistor 8b which detects light reflected by the vascular blood flow. Reference numeral 9 denotes a photoelectric pulse wave detection amplifier (AMP) which amplifies an output signal from the phototransistor 8b and outputs an analog pulse wave signal M. The LED 8a is connected with a light controller 18 which automatically varies the light quantity. On the other hand, the photoelectric pulse wave detection amplifier 9 is connected with a gain controller 19a which varies gain and a time constant controller 19b which varies a time constant of the amplifier 9. Also, the blood pressure measuring apparatus has an accelerometer 20 and body movement detection amplifier (AMP) 21 to detect body movements. They output an acceleration signal A. Reference numeral 10 denotes an A/D converter (A/D) which converts analog signals M, P, and A (not shown) into digital data D (not shown).

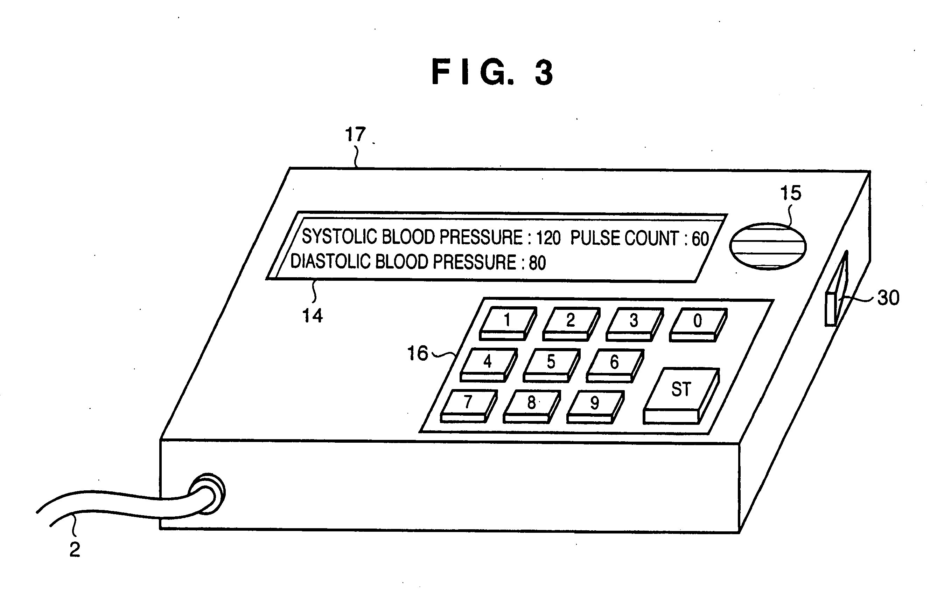

Reference numeral 11 denotes a controller (CPU) which performs main control of this blood pressure measuring apparatus. The CPU 11 has an adjustment pressure register 11a which stores adjustment pressure. Details of control will be described later. Reference numeral 12 denotes a ROM which stores a control program (such as that shown in Figure 3) executed by the CPU 11. Reference numeral 13 denotes a RAM which has a data memory, image memory, and the like. Reference numeral 14 denotes a liquid crystal display (LCD) which displays the contents of the image memory. Reference numeral 16 denotes a keyboard which allows the user to enter a measurement start command, set an adjustment pressure value, and so on. Reference numeral 15 denotes a buzzer which informs the user that the apparatus has sensed the activation of a key on a keyboard 16, that measurements have been done, and so on. Incidentally, although the adjustment pressure register 11a is installed in the CPU 11 in this example, an adjustment pressure storage unit may be installed in the RAM 13.

Figure 3 is an external perspective view of the blood pressure measuring apparatus according to the first embodiment. Reference numeral 17 denotes a main body of the sphygmomanometer, which contains components other than the cuff 1 and pulse wave sensor 8 in Figure 1. In Figure 3, the rubber tube (air tube) 2 includes a signal line for communication with the pulse wave sensor 8. It is connected to the cuff 1 and pulse wave sensor 8 (both not shown). The LCD display panel 14 is a dot-matrix display panel, and can display various information (e.g., characters, graphics, signal waveforms, and the like). Reference numeral 30 denotes a power switch. The keyboard 16 has a measurement start switch (ST) as well as a numeric keypad to enter a pressure value of the cuff and the like.

### <Attaching the cuff to a measurement site>

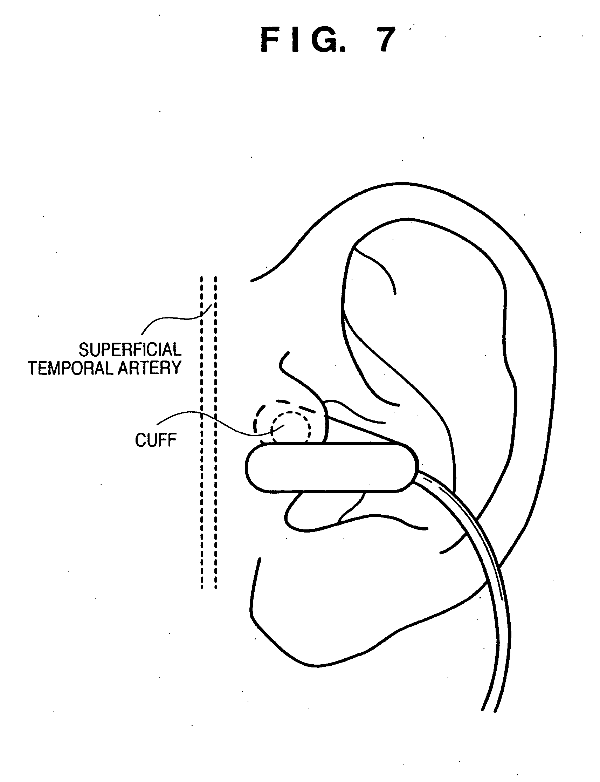

Figure 7 is a diagram showing a cuff attached to and around a tragus. Since a tragus and its surroundings are used as a measurement site, a measuring unit including the cuff is configured to squeeze the tragus by pinching it from both sides. Incidentally, since movements of the measurement site have the most impact on blood pressure values, the accelerometer 20 is preferably mounted near the mounting location of the measuring unit or mounted integrally with the measuring unit.

### <Equipment operation>

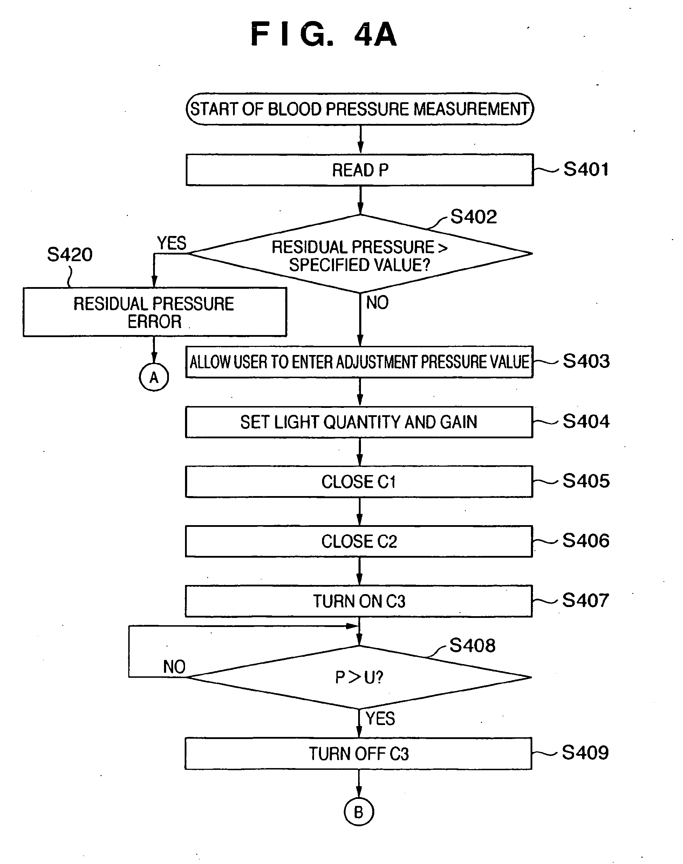

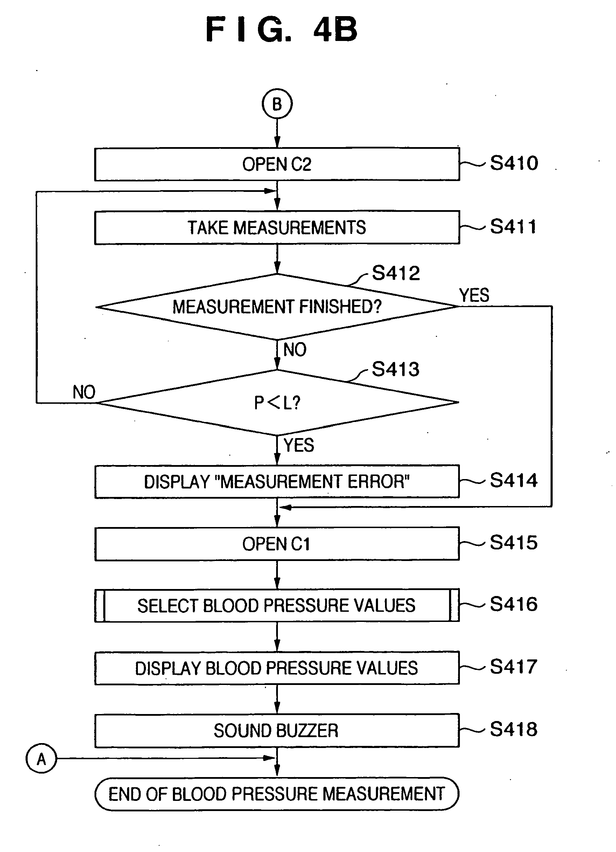

Figures 4A and 4B are operation flowcharts of the blood pressure measuring apparatus according to the first embodiment.

When the apparatus is powered on, it initializes itself by performing a self-diagnosis process (not shown). Subsequently, when the measurement start switch ST is pressed, the apparatus starts processing.

In Step S401, the apparatus reads the cuff pressure P. In Step S402, the apparatus compares residual pressure of the cuff 1 with a specified value. If the residual pressure exceeds the specified value, the apparatus displays "residual pressure error" on the LCD 14 in Step S420. If the residual pressure is not higher than the specified value, the apparatus allows the user in Step S403 to set a pressurization value (e.g., a value between 120 and 210 mmHg, which is higher than a systolic blood pressure) using the keyboard 16. In Step S404, the apparatus sets the light quantity and gain to predetermined values.

When the light quantity and gain have been set, the apparatus closes the quick exhaust valve 4 and slow exhaust valve 5 in Steps S405 and S406, respectively. In Step S407, the apparatus starts operating the pressure pump 3, and thereby starts pressurization (compression).

In Step S408, the apparatus determines whether or not the cuff pressure is higher than the pressurization value U set in Step S403. If P > U is not met, the apparatus continues pressurization. If P > U, the apparatus stops the pressure pump 3 in Step S409.

In Step S410, the apparatus opens the slow exhaust valve 5. This marks the start of a measurement step during depressurization (decompression). The cuff pressure starts to fall at a constant rate (e.g., 2 to 3 mmHg/sec). At the same time, a body movement detecting means (accelerometer) starts detecting acceleration and a first blood pressure determining means (photoplethysmography type) and second blood pressure determining means (pressure plethysmography type) start detecting pulse waves. Incidentally, the first blood pressure determining means illuminates blood vessels with light from the light-emitting element 8a, receives light reflected by the blood vessels using the light-receiving element 8b, and detects the light quantity (quantity of reflection which varies depending on the blood flow rate in the blood vessels) as a photoelectric pulse wave. At the same time, the second blood pressure determining means detects air pressure in the cuff, i.e., oscillation amplitude which varies with the amount of squeeze (air pressure which oscillates with oscillation of blood vessel walls corresponding to pulsation), as a pressure pulse wave using the pressure sensor. Meanwhile, in Step S411, various functional blocks perform data processing, and the apparatus measures systolic and diastolic blood pressures by the application of predetermined algorithms to a photoelectric pulse wave signal and pressure pulse wave signal. In Step S412, the apparatus determines whether or not a diastolic blood pressure value during depressurization has been detected. If both the diastolic blood pressure value measured from photoelectric pulse wave data and the diastolic blood pressure value measured from pressure pulse wave data have not been detected, the apparatus continues measurement. In Step S413, the apparatus determines whether or not the cuff pressure is lower than a predetermined value L (e.g., 40 mmHg). If P < L is not met, the cuff pressure is within a normal measuring range and thus the flow returns to Step S411. On the other hand, if P < L, the cuff pressure is already lower than the normal measuring range. Thus, if normal data is not obtained from either the photoelectric pulse wave signal or pressure pulse wave signal (e.g., if a determined systolic blood pressure is not higher than 40 mmHg), the apparatus displays "measurement error" on the LCD 14 in Step S414. In so doing, the apparatus additionally displays detailed information such as "signal failure during depressurization" if necessary. In Step S415, the apparatus opens the quick exhaust valve 4.

In Step S416; the apparatus selects either the systolic and diastolic blood pressure values determined from the photoelectric pulse wave or the systolic and diastolic blood pressure values determined from the pressure pulse wave depending on whether a value obtained by the accelerometer exceeds a predetermined value C. It is desirable to select the systolic and diastolic blood pressure values determined from the photoelectric pulse wave by determining that accurate blood pressure cannot be obtained from the pressure pulse wave due to body movements during measurement if the predetermined value C is exceeded, or select the systolic and diastolic blood pressure values determined from the pressure pulse wave if the predetermined value C is not exceeded. Incidentally, although the blood pressure values to be displayed are selected after deriving blood pressure values from each of the photoelectric pulse wave and pressure pulse wave, either the photoelectric pulse wave data or pressure pulse wave data may be selected before deriving blood pressure values.

In Step S417, the apparatus displays the selected systolic and diastolic blood pressure values on the LCD 14. In Step S418, the apparatus sounds a buzzer to inform the user of the end of measurements.

As described above, the sphygmomanometer according to this embodiment can objectively select proper blood pressure to be displayed out of blood pressure measurement results from the first blood pressure determining means of a photoplethysmography type and blood pressure measurement results from the second blood pressure determining means of a pressure plethysmography type based on the signal intensity from the accelerometer which is a body movement detecting means and using information as to whether a threshold corresponding to a predetermined acceleration has been exceeded as a judgment criterion. Incidentally, this embodiment is especially advantageous in measurements of a tragus and its surroundings, in which the effect of head movements cannot be ignored. Consequently, this embodiment can be applied easily to continuous measurement of blood pressure.

### (Second embodiment)

A second embodiment further has a function to calculate, using the CPU 11, periodic components of body movements from data produced by the accelerometer which is a body movement period calculating means. Thus, this embodiment makes it possible to effectively select blood pressure values for display output out of the blood pressure values determined from the photoelectric pulse wave and the blood pressure values determined from the pressure pulse wave in Step S416 for the following reasons.

When photoplethysmographic and pressure plethysmographic blood pressure measuring methods are compared based on measurement principles, the pressure plethysmographic method, which uses air for detection, is impervious to disturbing oscillation attributable to short-period (rapid) body movements because the disturbing oscillation is attenuated by air while the photoplethysmographic method is susceptible to short-period body movements. Thus, the pressure plethysmographic method is more desirable for blood pressure measurements in the presence of short-period (rapid) body movements of a lower magnitude than a predetermined value.

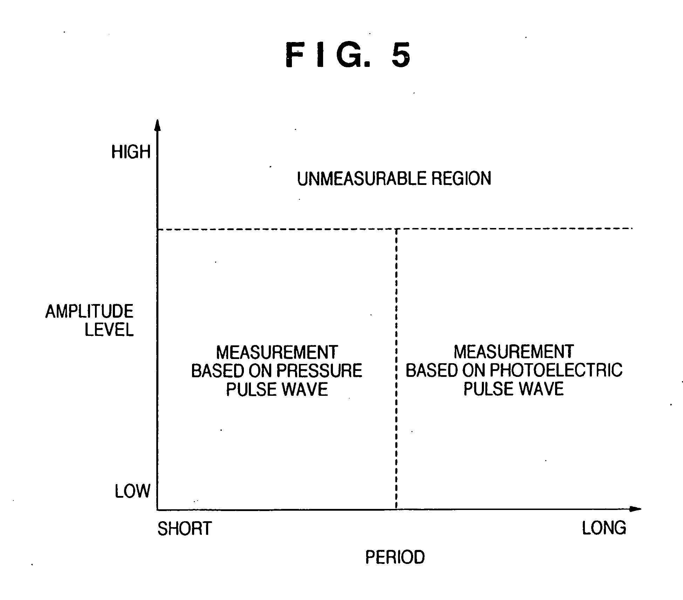

Figure 5 is a diagram showing exemplary choices of pulse waves based on a characteristic of body movements for a blood pressure measuring apparatus according to the second embodiment. Incidentally, although a photoelectric pulse wave is selected at a low noise level in this example, a pressure pulse wave may be detected more stably depending on the measurement site. In that case, a pressure pulse wave may be selected at a low noise level.

### (Third embodiment)

In a third embodiment, description will be given of a blood pressure measuring apparatus which can measure multiple sites at a time.

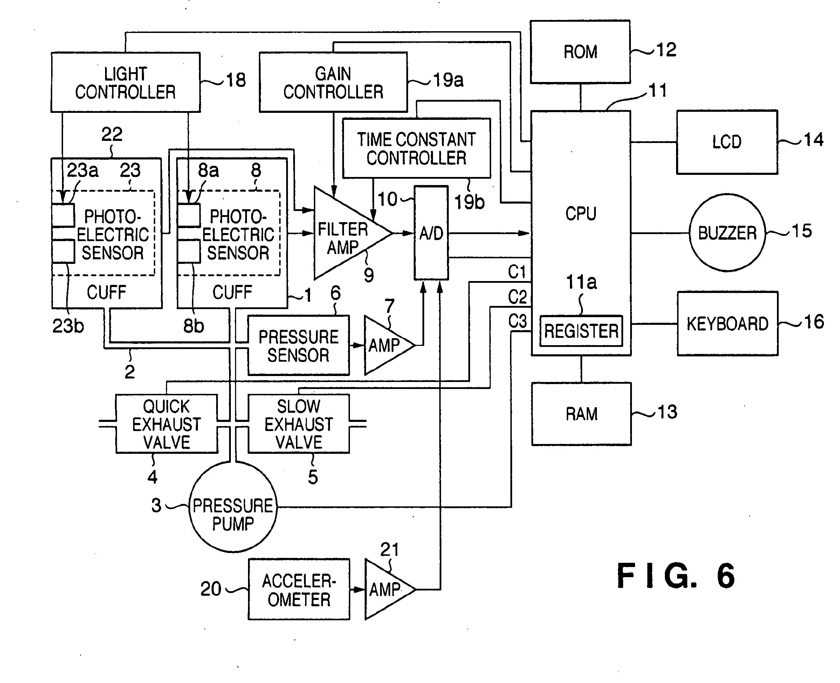

Figure 6 is an internal block diagram of the blood pressure measuring apparatus according to the third embodiment. Each cuff which pinches a tragus and/or its surroundings is equipped with a light-emitting unit (see Figure 6: LED 8a or 23a) and light-receiving unit (see Figure 6: phototransistor 8b or 23b). The two cuffs are configured to be pressurized by a single pressure pump 3 to measure blood pressure at multiple sites on and/or around a tragus, i.e., the front and back sides of the tragus, simultaneously. Incidentally, sensors based on different measurement principles (the pressure plethysmographic method and the like) may be used for blood pressure measurements. The rest of the configuration and operation is the same as the first and second embodiments, and thus description thereof will be omitted.

It is known that blood vessels (arterioles) in and/or around the tragi are located in close vicinity to blood vessels in the brain, and it is considered that changes in blood pressure resulting from intracerebral causes can be measured. On the other hand, around the tragi, there are not only blood vessels (arterioles) in the ear cartilage (mainly tragi), but also arteries (superficial temporal artery) directly connected to the heart. This offers the advantage of being able to measure blood pressures carrying different pieces of information (blood pressure attributable to the brain and blood pressure attributable to the heart) simultaneously around a tragus using a small apparatus. The blood pressure measuring apparatus according to this embodiment makes it possible to objectively select the most probable result out of blood pressure measurement results produced by multiple methods, based on a characteristic of body movements during a period of blood pressure measurement, and thereby take high-accuracy blood pressure measurements around a tragus.

## Claims

1. A blood pressure measuring apparatus comprising:
a cuff (1) which is configured to be attached to and around an external ear;
a pressure pulse wave detector (6) configured to detect a pressure pulse wave in a part squeezed by said cuff and a photoelectric pulse wave detector (8) configured to detect a pulse wave in a part squeezed by said cuff, the pressure pulse wave detector (6) and the photoelectric pulse wave detector (8) being affected differently by a characteristic of body movements;
body movement detecting means (20) configured to detect body movements during measurement; and
blood pressure value deriving means (11) configured to derive systolic and diastolic blood pressure values based on a detected pulse wave;
**characterized in that**
said body movement detecting means is an accelerometer (20);
pulse wave selecting means (11) are provided configured to select a pulse wave detected by one of said pressure pulse wave detector (6) and said photoelectric pulse wave detector (8) based on a characteristic of body movements detected by said accelerometer (20);
the blood pressure value deriving means are configured to derive the systolic and diastolic blood pressure values on the basis of the detected pulse wave selected by said pulse wave selecting means; and
said pulse wave selecting means (11) are configured to select the pressure pulse wave detected by said pressure pulse wave detector (6) when a value obtained by said accelerometer (20) does not exceed a predetermined value and selects the photoelectric pulse wave detected by said photoelectric pulse wave detector (8) when the value obtained by said accelerometer exceeds said predetermined value.

2. A blood pressure measuring method using a cuff (1) which is attached to and around an external ear;
the method comprising the steps of:-
detecting a pressure pulse wave in a part squeezed by said cuff by means of a pressure pulse wave detector (6) and detecting a pulse wave in said part by means of a photoelectric pulse wave detector (8), the pressure pulse wave detector (6) and the photoelectric pulse wave detector (8) being affected differently by a characteristic of body movements;
detecting body movements during measurement; and deriving systolic and diastolic blood pressure values baaed on the detected pulse wave;
**characterized in that**
said body movement is detected by an accelerometer (20); and
the method includes the step of selecting a pulse wave detected by one of said pressure pulse wave detector (6) and said photoelectric pulse wave detector (8) on the basis of the characteristic of body movements detected by said accelerometer (20), and performing said step of deriving systolic and diastolic blood pressure values on the basis of the pulse wave so selected;
the pulse wave selected in the selecting step being the pressure pulse wave detected by said pressure pulse wave detector (6) when a value obtained by said accelerometer (20) does not exceed a predetermined value and being the photoelectric pulse wave detected by said photoelectric pulse wave detector (8) when the value obtained by said accelerometer exceeds said predetermined value.

## Patentansprüche

1. Blutdruckmessvorrichtung mit:
einer Manschette (1), die dazu aufgebaut ist, an einer und um eine Ohrmuschel angebracht zu sein;
einem Druckpulswellendetektor (6), der dazu angepasst ist, eine Druckpulswelle in einem von der Manschette gedrückten Teil zu erfassen, und einem fotoelektrischen Pulswellendetektor (8), der dazu angepasst ist, eine Pulswelle in einem von der Manschette gedrückten Teil zu erfassen, wobei der Druckwellenpulsdetektor (6) und der fotoelektrische Pulswellendetektor (8) durch eine Charakteristik von Bewegungen des Körpers unterschiedlich beeinflusst werden;
einer Einrichtung (20) zur Erfassung der Körperbewegung, die dazu aufgebaut ist, Körperbewegungen während der Messung zu erfassen; und
einer Einrichtung (11) zur Ableitung des Blutdruckwerts, die dazu angepasst ist, systolische und diastolische Blutdruckwerte auf der Grundlage einer erfassten Pulswelle abzuleiten;
**dadurch gekennzeichnet, dass**
die Einrichtung zur Erfassung der Körperbewegung ein Beschleunigungsmesser (20) ist;
eine Einrichtung (11) zur Auswahl der Pulswelle vorgesehen ist, die dazu angepasst ist, eine Pulswelle, die entweder durch den Druckpulswellendetektor (6) oder den fotoelektrischen Pulswellendetektor (8) erfasst wird, auf der Grundlage einer Eigenschaft von Körperbewegungen auszuwählen, die von dem Beschleunigungsmesser (20) erfasst werden;
wobei die Einrichtung zur Ableitung des Blutdruckwerts dazu angepasst ist, die systolischen und diastolischen Blutdruckwerte auf der Grundlage der erfassten Pulswelle abzuleiten, die von der Einrichtung zur Auswahl der Pulswelle ausgewählt ist; und
wobei die Einrichtung (11) zur Auswahl der Pulswelle dazu aufgebaut ist, die von dem Druckpulswellendetektor (6) erfasste Druckpulswelle auszuwählen, wenn ein Wert, der von dem Beschleunigungsmesser (20) erhalten wird, einen vorab festgelegten Wert nicht übersteigt, und die von dem fotoelektrischen Pulswellendetektor (8) erfasste fotoelektrische Pulswelle auszuwählen, wenn der Wert, der von dem Beschleunigungsmesser erfasst wird, den vorab festgelegten Wert übersteigt.

2. Verfahren zum Messen eines Blutdrucks unter Verwendung einer Manschette (1), die an einer und um eine Ohrmuschel angebracht ist, wobei das Verfahren folgende Schritte aufweist:
Erfassen einer Druckpulswelle in einem Teil, der von der Manschette gedrückt wird, mittels eines Druckpulswellendetektors (6) und Erfassen einer Pulswelle in diesem Teil mittels eines fotoelektrischen Pulswellendetektors (8), wobei der Druckpulswellendetektor (6) und der fotoelektrische Pulswellendetektor (8) durch eine Eigenschaft von Körperbewegungen unterschiedlich beeinflusst werden;
Erfassen von Körperbewegungen während der Messung; und
Ableiten von systolischen und diastolischen Blutdruckwerten auf der Grundlage der erfassten Pulswelle;
**dadurch gekennzeichnet, dass**
die Körperbewegung durch einen Beschleunigungsmesser (20) erfasst wird; und
das Verfahren den Schritt der Auswahl einer Pulswelle, die durch entweder den Druckpulswellendetektor (6) oder den fotoelektrischen Pulswellendetektor (8) erfasst wird, auf der Grundlage der Charakteristik von Körperbewegungen, die von dem Beschleunigungsmesser (20) erfasst werden, und der Durchführung des Schritts der Ableitung systolischer und diastolischer Blutdruckwerte auf der Grundlage der so ausgewählten Pulswelle umfasst;
wobei die Pulswelle, die in dem Auswahlschritt ausgewählt ist, die von dem Druckpulswellendetektor (6) erfasste Druckpulswelle ist, wenn ein Wert, der von dem Beschleunigungsmesser (20) erfasst wird, einen vorab festgelegten Wert nicht übersteigt, und die von dem fotoelektrischen Pulswellendetektor (8) erfasste fotoelektrische Pulswelle ist, wenn der Wert, der von dem Beschleunigungsmesser erfasst wird, den vorab festgelegten Wert übersteigt.

## Revendications

1. Appareil de mesure de pression sanguine comprenant :
une manchette (1) qui est configurée pour être fixée sur et autour d'une oreille externe ;
un détecteur d'onde pulsée de pression (6) configuré pour détecter une onde pulsée de pression dans une partie comprimée par ladite manchette et un détecteur d'onde pulsée photoélectrique (8) configuré pour détecter une onde pulsée dans une partie comprimée par ladite manchette, le détecteur d'onde pulsée de pression (6) et le détecteur d'onde pulsée photoélectrique (8) étant affectés différemment par une caractéristique des mouvements du corps ;
des moyens de détection du mouvement du corps (20) configurés pour détecter les mouvements du corps pendant la mesure ; et
des moyens de dérivation de valeur de pression sanguine (11) configurés pour dériver les valeurs de pression sanguine systolique et diastolique en fonction d'une onde pulsée détectée ;
**caractérisé en ce que** :
lesdits moyens de détection du mouvement du corps sont un accéléromètre (20) ;
des moyens de sélection d'onde pulsée (11) sont configurés pour sélectionner une onde pulsée détectée par l'un parmi ledit détecteur d'onde pulsée (6) et ledit détecteur d'onde pulsée photoélectrique (8) en fonction d'une caractéristique des mouvements du corps détectés par ledit accéléromètre (20) ;
les moyens de dérivation de valeur de pression sanguine sont configurés pour dériver les valeurs de pression sanguine systolique et diastolique en fonction de l'onde pulsée détectée sélectionnée par lesdits moyens de sélection d'onde pulsée ; et
lesdits moyens de sélection d'onde pulsée (11) sont configurés pour sélectionner l'onde pulsée de pression détectée par ledit détecteur d'onde pulsée de pression (6) lorsqu'une valeur obtenue par ledit accéléromètre (20) ne dépasse pas une valeur prédéterminée et sélectionne l'onde pulsée photoélectrique détectée par ledit détecteur d'onde pulsée photoélectrique (8) lorsque la valeur obtenue par ledit accéléromètre dépasse ladite valeur prédéterminée.

2. Procédé pour mesurer la pression sanguine à l'aide d'une machette (1) qui est fixée sur et autour d'une oreille externe ;
le procédé comprenant les étapes consistant à :
détecter une onde pulsée de pression dans une partie comprimée par ladite manchette au moyen d'un détecteur d'onde pulsée de pression (6) et détecter une onde pulsée dans ladite partie au moyen d'un détecteur d'onde pulsée photoélectrique (8), le détecteur d'onde pulsée de pression (6) et le détecteur d'onde pulsée photoélectrique (8) étant affectés différemment par une caractéristique des mouvements du corps ;
détecter les mouvements du corps pendant la mesure ; et
dériver les valeurs de pression sanguine systolique et diastolique en fonction de l'onde pulsée détectée ;
**caractérisé en ce** qui :
ledit mouvement du corps est détecté par un accéléromètre (20) ; et
le procédé comprend l'étape consistant à sélectionner une onde pulsée détectée par l'un parmi ledit détecteur d'onde pulsée de pression (6) et ledit détecteur d'onde pulsée photoélectrique (8) en fonction de la caractéristique des mouvements du corps détectés par ledit accéléromètre (20) et réaliser ladite étape consistant à dériver les valeurs de pression sanguine systolique et diastolique en fonction de l'onde pulsée ainsi sélectionnée ;
l'onde pulsée sélectionnée à l'étape de sélection étant l'onde pulsée de pression détectée par ledit détecteur d'onde pulsée de pression (6) lorsqu'une valeur obtenue par ledit accéléromètre (20) ne dépasse pas une valeur prédéterminée et étant l'onde pulsée photoélectrique détectée par ledit détecteur d'onde pulsée photoélectrique (8) lorsque la valeur obtenue par ledit accéléromètre dépasse ladite valeur prédéterminée.
